(19) Europäisches Patentamt

European Patent Office

Office européen des brevets

(11) EP 0 739 484 B1

(12) **EUROPEAN PATENT SPECIFICATION**

(45) Date of publication and mention of the grant of the patent:
**10.12.1997 Bulletin 1997/50**

(21) Application number: **94905004.1**

(22) Date of filing: **21.01.1994**

(51) Int Cl.6: **G01N 33/02**, G01N 33/24, G01N 23/22

(86) International application number:
**PCT/DK94/00038**

(87) International publication number:
**WO 94/17404 (04.08.1994 Gazette 1994/18)**

(54) **A METHOD OF DETECTING THE EARTH CONTENT IN BEETS**

VERFAHREN ZUM NACHWEIS DES ERDGEHALTES VON RÜBEN

PROCEDE POUR DETERMINER LA TENEUR EN TERRE DE BETTERAVES

(84) Designated Contracting States:
**AT BE CH DE ES FR GB GR IE IT LI NL PT SE**

(30) Priority: **27.01.1993 DK 91/93**

(43) Date of publication of application:
**30.10.1996 Bulletin 1996/44**

(73) Proprietor: **DANISCO A/S**
**1411 Copenhagen K. (DK)**

(72) Inventors:
• **JORGENSEN, Lars, Bo**
**DK-4800 Nyk bing F (DK)**
• **FRYDENDAL, Ib**
**DK-4900 Nakskov (DK)**
• **THOMASSEN, Jesper**
**DK-4913 Horslunde (DK)**
• **HANSEN, Ole**
**DK-4900 Nakskov (DK)**

(74) Representative: **Siiger, Joergen et al**
**c/o Chas. Hude**
**H.C. Andersens Boulevard 33**
**1553 Copenhagen V (DK)**

(56) References cited:
**US-A- 4 992 667**

• **PROCEEDINGS OF THE 14TH CONFERENCE 1992 OF THE AUSTRALIAN SOCIETY FOR SUGAR CANE TECHNOLOGISTS, Held at Mackay, Queensland from 28th April to 1st May 1992, P.G. ATHERTON et al.: "The Determination of Soil in Cane".**

**Description**

Technical Field

The invention relates to a method of detecting the earth content in agricultural crops, such as beets mixed with earth by a measuring of the $\gamma$-radiation of the earth, the counting number of the measured $\gamma$-radiation being corrected for differences in different types of earth, having different contents of potassium, uranium and thorium.

Background Art

An article by P.G. Atherton in Annual Report Sugar Research Council (Australia) 1989/90 p. 58 describes an employment of the $\gamma$-radiation emitted by isotopes in the earth as a measurement of the earth content in sugar cane deliveries. Tests have shown that 0 to 5 % by weight of earth in the sugar cane deliveries can be determined with a statistic accuracy of 0.8% at the 95% confidence level. Based on measurements performed on 16 different earth types Atherton indicates that individual calibrations between $\gamma$-counting numbers and the earth percentage can be necessary because the earth types contain different amounts of isotopes.

Brief Description of the Invention

The object of the invention is to provide a simple method of correcting for the content of different isotopes in the earth.

The method according to the invention is characterised by the counting number being corrected for the content in the earth of humus by only compensating for the content of potassium in the earth. As a result it is very easy to compensate for the content of different isotopes in the earth, as nothing but the content of humus in the earth must be determined.

Furthermore it is according to the invention possible to correct for the content of humus in the earth by compensating for the content of potassium in the earth by utilizing that humus contains a particularly high amount of uranium compared to the amount of potassium.

The correction for the content of potassium may according to a first approximation be performed by the counting number Int being corrected by the factor

K/overK

where Int is the counting number in almost the entire energy range, K is the counting number in the range containing the highest amount of potassium, and overK is the counting in the energy range thereabove. As a result, the variation of the measuring results is reduced.

The correction can be improved by correcting for the type of humus by means of a step function having a soft transition. As a result, the variation of the measuring results is further reduced.

Brief Description of the Drawing

The invention is explained in greater detail below with reference to the accompanying drawings, in which

Fig. 1 illustrates an apparatus for removing beet samples and detecting the earth content by the method according to the invention,

Fig. 2 illustrates the average spectrum of energy for 20 earth samples,

Fig. 3 illustrates the counting numbers of the different earth samples before and after the correction for the content of potassium,

Fig. 4 illustrates the spectrum of energy for sample No. 2,

Fig. 5 illustrates the spectrum of energy for sample No. 7,

Fig. 6 illustrates the spectrum of energy for sample No. 15,

Fig. 7 illustrates the counting numbers of the 20 earth samples after the correction for the content of different types of humus, and

Fig. 8 illustrates a step function for correcting for different types of humus.

Best Mode for Carrying Out the Invention

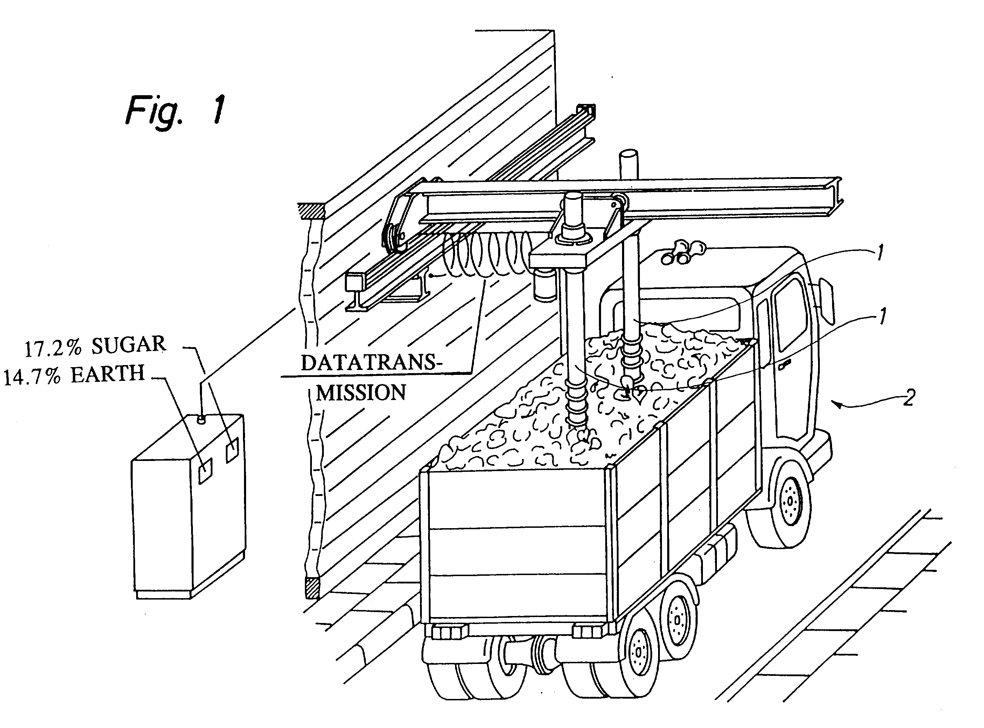

The apparatus shown in Fig. 1 comprises a plurality of boring tubes 1 to be bored into a load of beets on a lorry 2. Each tube 1 comprises a detector capable of measuring the γ-radiation from the earth in the beet load. As the different earth types emit different γ-radiations per mass unit, it is necessary to correct for the type of earth so as to obtain a sufficient accuracy. Radioactive substances (isotopes) in earth, such as uranium, potassium, and thorium emit γ-rays with different energies.

The on-line measuring apparatus shown in Fig. 1 is used for measuring the earth percentage in loads of beets on a lorry 2. When the lorry 2 is stopped, a boring tube 1 is bored into the load of beets. The boring tube 1 comprises a detector measuring the γ-radiation from the earth in the load. After measuring for about 1 minute, the boring tube 1 is retracted and the beets are unloaded. The detector comprises an NaI-crystal. When this NaI-crystal meets a γ-quantum, said quantum may be absorbed by the NaI-crystal emitting a high or low amount of its energy. A γ-quantum emitted by the isotope potassium-40 contains for instance an energy of 1.46 MeV. The emission of such a quantum cannot therefore exceed 1.46 MeV. In connection with a partial absorption, the emitted energy is lower. Although the energy of the quantum is only partially absorbed, the remaining energy can nevertheless be absorbed by the NaI-crystal. When a quantum emits energy to the crystal, said emission involves emission of photons. The detector utilizes that the energy of the emitted photons depends in an unambiguous manner on the absorbed energy of the γ-quantum. When the emission of light from the NaI-crystal is quantitatively measured, it is possible to determine the energy absorbed from a γ-quantum.

The measuring period can be almost halved by two boring tubes being used.

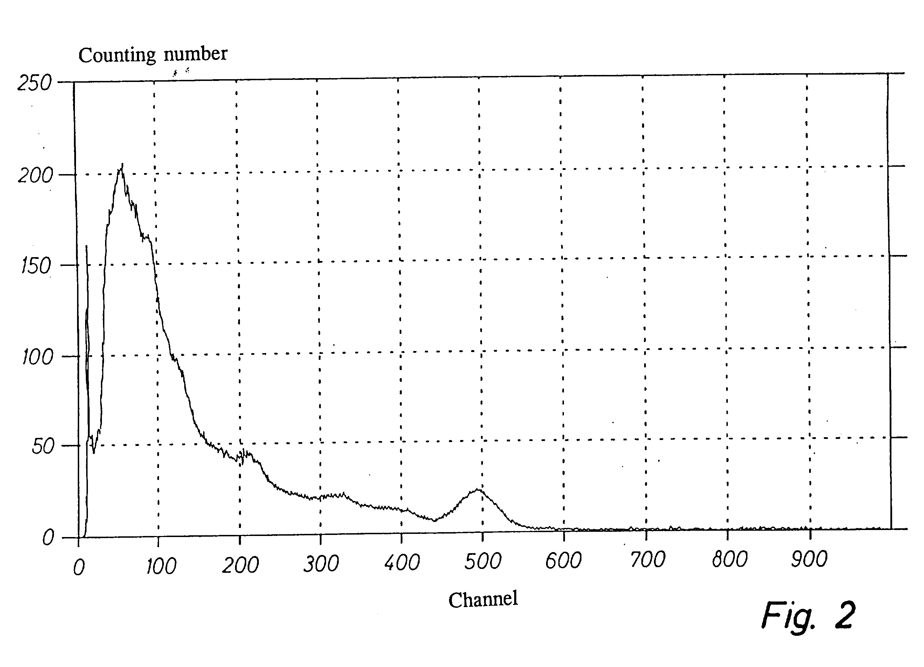

The measuring apparatus, cf. Fig. 1 measures in 1024 channels corresponding to 1024 different energies. The energy is proportional to the channel number. Channel No. 1 corresponds to an energy of 3 KeV, and channel No. 1024 corresponds to an energy of 3.07 MeV. Reconsidering potassium-40 with an energy of 1.46 MeV, a high amount of this isotope in the material to be analyzed results in a peak in the spectrum of energy adjacent channel No. 487. This appears from Fig. 2 showing a spectrum of energy representing the average value of 20 different types of earth. The potassium peak appears in the spectrum adjacent channel No. 487. The very low energies matching channels 1 to 25 are very sensitive to noise. A noise peak appears on the channels 10 to 15. The very high peak adjacent channel 60 originates partly from isotopes emitting quanta in this energy range and partly from quanta of higher energies and only having released some of their energy to the NaI-crystal. Both uranium and thorium emit energy quanta of a low energy while decaying to elements of a lower number. The emission of quanta having released an amount of their energy corresponds, however, more or less to a large number of secondary energy emissions within the low energy range.

In the beet load the detector is surrounded by a large amount of beets and earth. The background radiation is therefore very low compared to the radiation from the earth. In this manner an almost pure signal is received from the earth. The detector detects the signal in a spherical area inside the beet load, but as both beets and earth attenuate the γ-radiation, the radiations emitted by the earth closest to the detector are strongest. The area detected by the detector is considered to be semi-infinite. When the density of the earth in the load is higher, the halving distance of the γ-radiation containing a specific energy is lower. Despite this fact, the number of quanta hitting the detector increases with the density of earth. If desired, it is possible to correct for background radiation.

The total number of quanta transmitted in equally large time intervals from the same amount of earth increases in the order sand, sandy clay, and heavy clay. Heavy types of clay would transmit almost twice as many γ-quanta as sand without the background radiation. A utilization of the total number of transmitted γ-quanta per time unit as a direct measurement for the density of the earth in a beet load is therefore subject to a high uncertainty.

The difference between sand and heavy clay is not only the total intensity, but the spectra differ too. The spectrum of each type of earth can be considered the finger-print of each type of earth. These finger-prints contain useful information. The low energies in the measured spectrum have to a certain degree been compensated for by partially absorbed quanta with higher energies and by secondary absorptions. The intermediary interval provide, however, so perfect measurements that they can be used for characterising the types of earth. In this manner it is possible to compensate for different radiations from different types of earth.

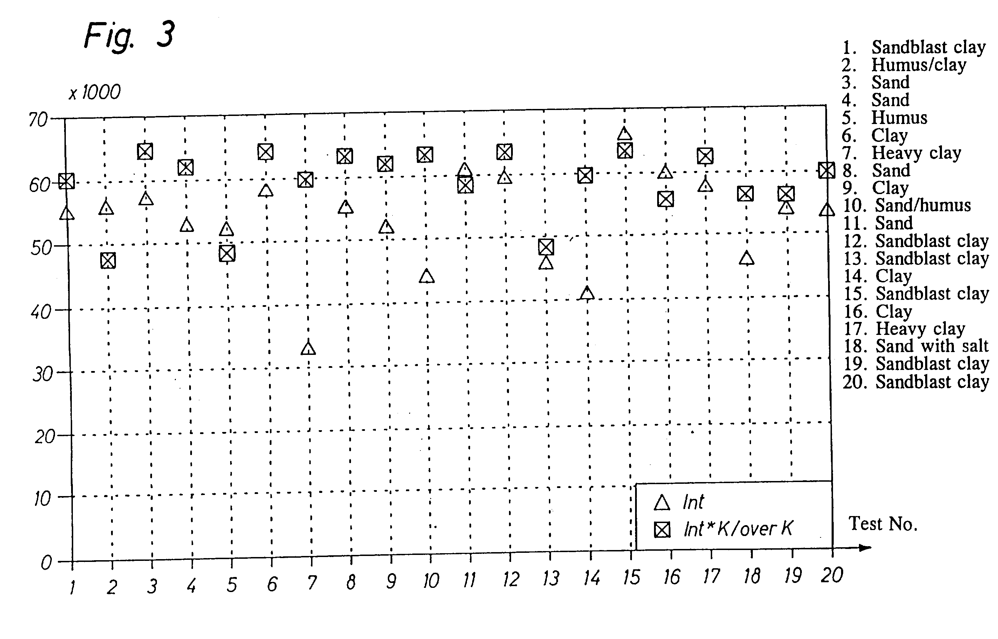

The purer intervals of the spectrum with higher energies are not attractive due to the relatively low counting numbers in these intervals. The number of quanta transmitted by a radioactive source for equally long periods is substantially poison-distributed. It means that the variation of measured quanta corresponds to the square root of the average value. Low counting numbers present therefore a relatively large variation (variation/average value). The relative variation is interesting because the measuring technique is based on almost perfect measurements of the γ-radiation of the earth. Irrespective of corrections, the final number must in an unambiguous manner depend on the earth percentage. One of the most interesting isotopes in the spectrum of energy is potassium-40. A summarizing of the counting numbers in the channels with the strongest radiations from this isotope is utilized in a first embodiment for earth type correction, where the counting number Int is multiplied with the correction factor K/overK, which results in

$$\text{Int} \bullet K/overK$$

where Int is the counting number in the energy range of 225 keV to 3045 keV, K is the counting number in the energy range of 1290 keV to 1590 keV, and overK is the counting number in the energy range of 1650 keV to 3045 keV. This embodiment ensures a considerable improvement compared to the total counting number alone, cf. Fig. 3, which clearly shows that the variation is reduced by a multiplication with the correction factor. The total counting number of the 20 earth samples examined showed a relative variation of 15%. It is possible to reduce the variation to 9.6% by means of the correction factor.

Fig. 3 shows furthermore that after multiplication with the correction factor the earth samples 2, 5 and 13 are nevertheless placed considerably lower than the remaining samples. The samples 2, 5, and 13 are all types of humus. Accordingly a correction for types of humus must be performed. It appears from Fig. 3, that the three types of humus do not differ essentially relative to one another. If humus can be identified, it must therefore be possible to compensate therefor.

Experience has taught that potassium is less active in humus than in other types of earth. The relationship between the integrated counting number in the low energy range (150 to 450 KeV) divided by the counting number in the potassium range (1290 to 1590 keV) provides therefore a good indication of whether the earth contains humus. The above locations of the two energy ranges (windows) result in a limit of about 3.85. When the relationship between the countings in the two ranges, i.e. 540 to 630 KeV and 1290 to 1550 KeV, exceeds 3.85, the sample must be considered containing humus. On the other hand when the relationship is lower than 3.85, it is a question of other types of earth. By such an indication, uranium is an important factor because uranium decays to Bi-314 producing a strong peak with a centre in 609.3 KeV. A high counting number in this range can be due to a high content of uranium.

A correction for types of humus is performed by means of a soft step function of the formula

$$f(x) = 1 + h - h/(\exp a\,(x - x_0)) + 1)$$

illustrated in Fig. 8. This function is advantageous in changing from the value 1 to the level $1 + h$ when $x = x_0$. $h$ is the difference between the level before and after the change, $x_0$ is the centre of the change, and $a$ is a constant determining the narrowness of the change interval.

Together with the limit of 3.85 this function is used to separate humus from the remaining types of earth so as to compensate for humus. The total correction factor to be multiplied with Int is

$$[1 + h - h/(\exp(a(x - x_0)) + 1)]\ K/overK$$

The constants have been found by way of tests and are

$$h = 0{,}35$$

$$a = 20$$

$$x_0 = 3{,}85.$$

This embodiment has been tested on 20 types of earth, and the variation divided by the average number is 4,6%. Thus the variation has been considerably reduced.

Example

This example deals with three different types of earth. The sample numbers are

2: humus/clay

7:     sand
15:    heavy clay.

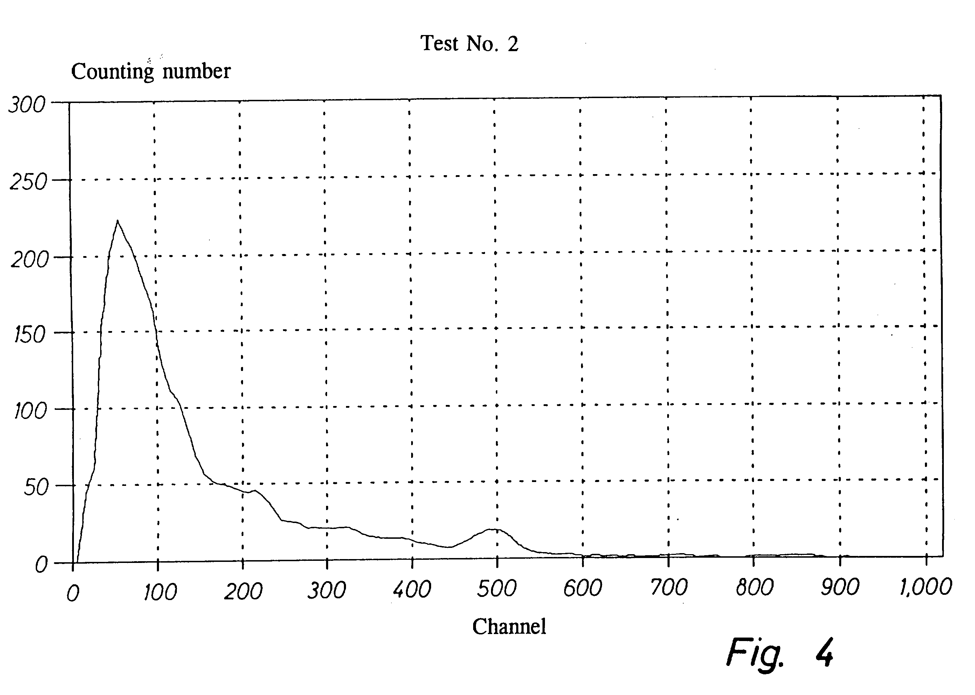

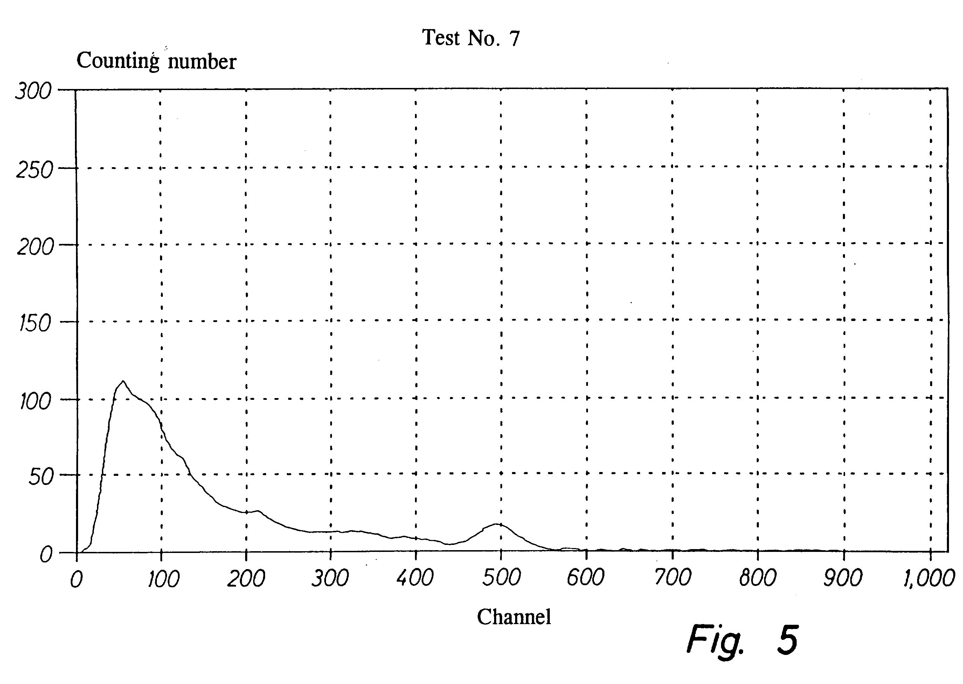

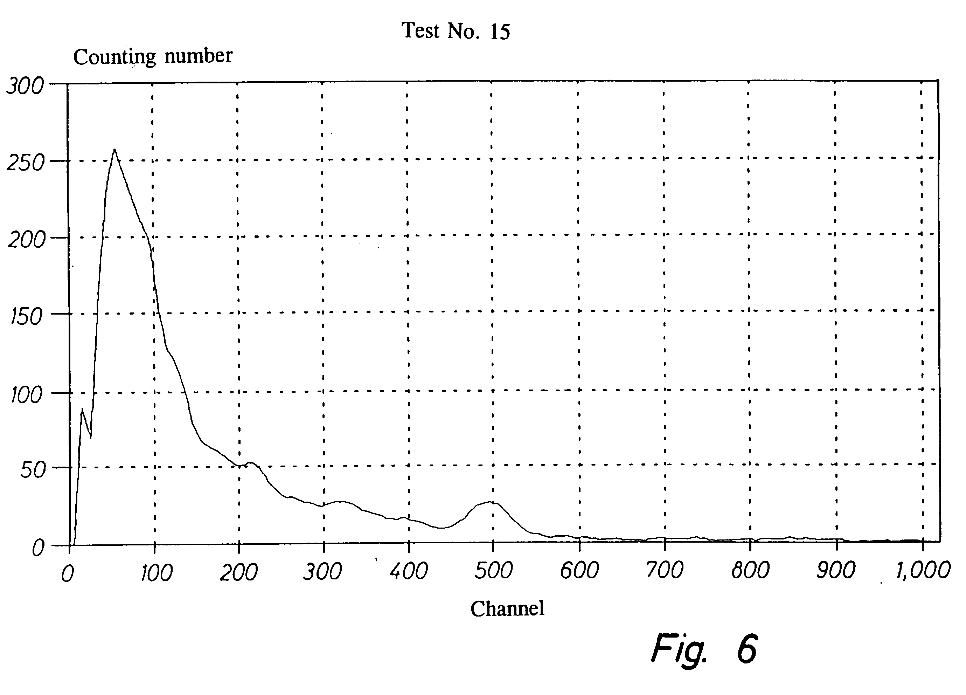

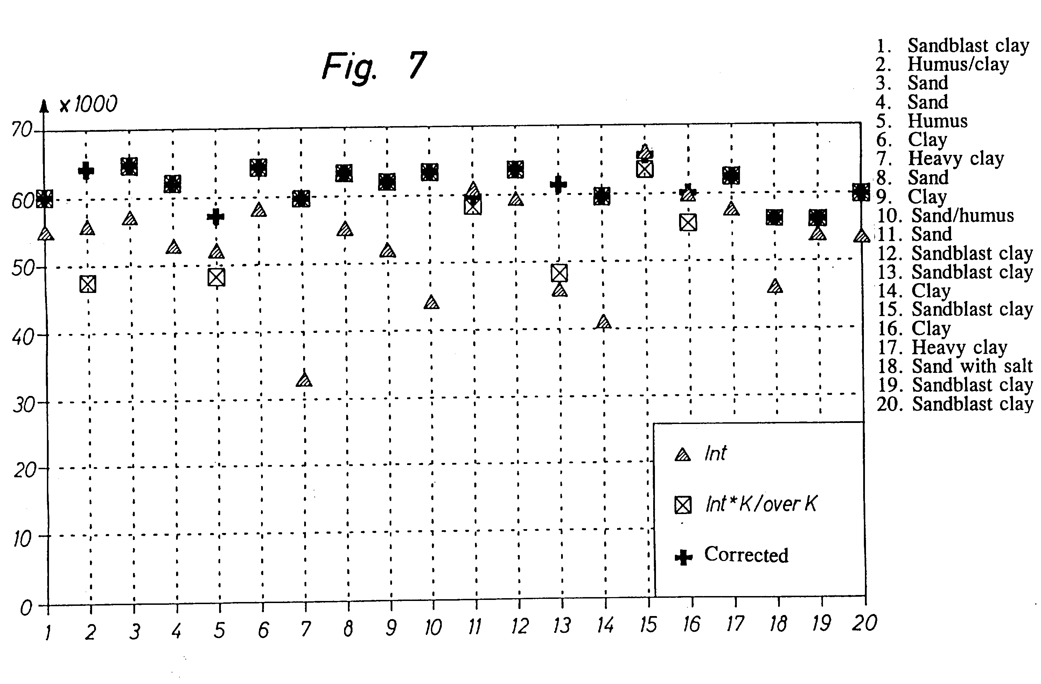

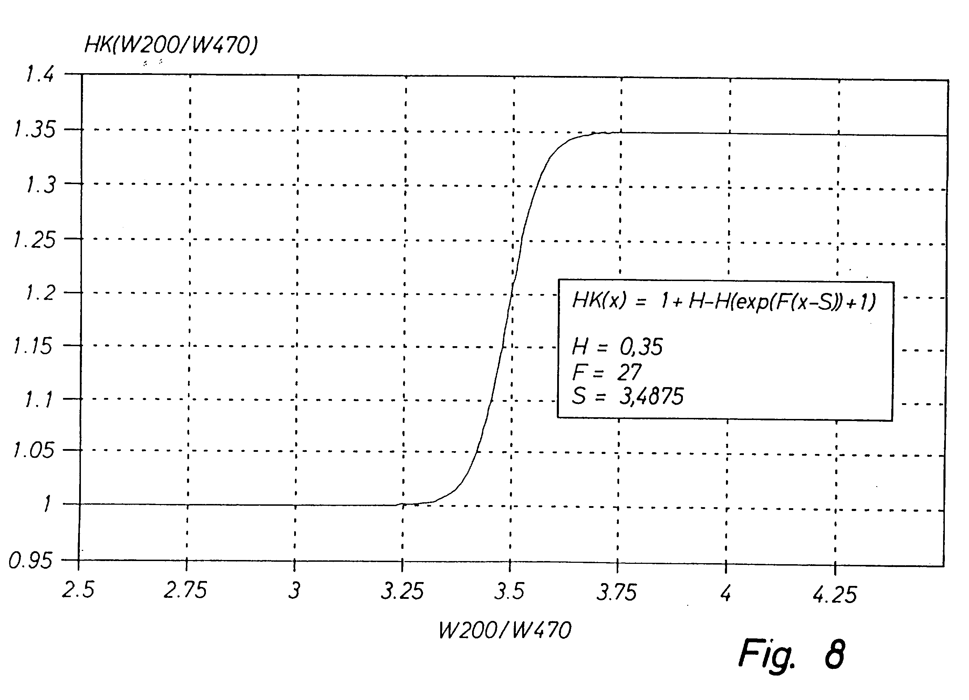

The spectrum of energy of these three types of earth appears from Figs. 4 to 6. The counting numbers are as follows:

| Sample No. | | 2 | 7 | 15 |
|---|---|---|---|---|
| | [Energy range] | | | [Counting number] |
| Int | (225-3045 keV) | 19992 | 11815 | 23169 |
| K | (1335-1590 keV) | 1298 | 1084 | 1701 |
| overK | (1650-3045 keV) | 545 | 214 | 633 |
| W200 | (540-630 keV) | 5673 | 3366 | 6688 |
| W480 | (1290-1590 keV) | 1378 | 1128 | 1788 |
| calculated values . ... | | | | |
| Int • K/overK | | 47614 | 59848 | 63469 |
| W200/W480 | | 4,12 | 2,98 | 3,74 |
| Correction factor | | 1,348 | 1,000 | 1,035 |
| Comp. • Int • K/overK | | 64184 | 59848 | 65690 |

It appears that sample No. 2 is a type of humus. Despite the different types of earth, the deviation from the highest value 65690 for sample No. 15 to the lowest value 59848 for sample No. 7 amounts only to 9.2% of the average value. When this embodiment is used on the 20 samples of earth, the variation of the results is reduced to 4.6% of the average value. Fig. 7 illustrates the following for all 20 samples of earth

-      the sum of the countings from the energy range 225 keV to 3045 keV
-      the sum of the countings corrected by Int • K/overK
-      the sum of the countings corrected by K/overK and by the correction for humus.

The variation of the results is acceptable.

A demand exists for a higher counting number in all energy ranges, as the necessary measuring time would thereby be reduced. A higher counting number can be obtained by summing up over large intervals of the spectrum or by placing the windows in which said summing up is performed in intervals presenting a higher counting number per channel. It appears from the above table that the lowest counting numbers are found in the window "overK". This window cannot be extended as it would then be influenced by the radiation from potassium. Accordingly another embodiment must be developed which only utilizes windows in the range from and including potassium and further down. Here it is possible to utilize that uranium and thorium also emit quanta of lower energies during their decay. A correction for humus according to such an embodiment renders it possible to reduce the measuring period.

The factor $W\ 470^2/W87$ acts like Int • K/overK which means that the relative variation of the measurings is almost the same on the 20 samples after the correction by means of this factor because said factor (the correction factor) also presents low values for humus. W470 is the potassium peak and W87 is the sum of the channels covering energies from keV 225 to 300 keV. In this range Ac - 228 (daughter of Thorium-232) and Ra - 226 (daughter of uranium-238) are found. The range is furthermore influenced by secondary absorptions on other isotopes, which results in a slight blurring of the radiations from uranium and thorium. $W\ 470^2/W87$ is of the same nature as Int • K/overK because the potassium window has apparently more weight than in the previous embodiment when W470 is raised to the 2nd power. The effect of uranium and thorium is, however, moderated as the counting numbers of the high energies are not "pure", but instead W87 from very low energies also depending on other conditions.

Example of attenuation of γ-radiation in a load of beets.

$m_R$   is the mass of clean beets in the load
V      is the volume of the entire load (a constant)
J%     is the earth percentage calculated at $m_J/m_R$

In other words, the earth percentage is calculated as a percentage of the clean beets and not as a percentage of the total mass.

The density of clean beets in the load is therefore:

$$\rho_R = \frac{m_R}{V}$$

The density of the entire load is determined by

$$\rho_B = \frac{(1+J\%)\,m_R}{V} = (1+J\%)\,\rho_R$$

The density of the earth alone in the volume of the entire load is

$$\rho_J = \frac{J\%\,m_R}{V} = J\%\,\rho_R$$

$\rho_B$ representing the density of attenuating material, and $\rho_J$ representing the density of $\gamma$-radiating material.

The area of a spherical shell of a radius r is found at $4\pi r^2 \Delta r$. The volume of a spherical shell of the thickness $\Delta r$ is approximately

$$\Delta V = 4\pi r^2 \Delta r$$

The mass of radiating material in this spherical shell is then

$$\Delta m = \Delta V \rho_J$$

$$= 4\pi r^2 \rho_J \Delta r$$

The number of transmitted quanta from the mass $\Delta m$ in the period $\Delta t$ is $\Delta I_0 = E_0 \Delta m \Delta t$, where $E_0$ is the counting number from the earth measured by the detector per time unit and per mass unit of earth. $E_0$ depends on the detector.

At the distance r from the earth with the mass $\Delta m$ and during the period $\Delta t$ the following unattenuated signal is measured by the detector:

$$\Delta I_1\,(r) = \frac{\Delta I_0}{r^2}$$

the solid angle "seen" by a radiation-transmitting element being inversely proportional to the square on the distance. This signal is attenuated across the distance to the detector. This distance is $r\text{-}r_0$, where $r_0$ is the radius of the detector. The measured signal is consequently:

$$\Delta I_2\,(r) = \frac{\Delta I_0}{r^2}e^{-\mu(r-r_0)}$$

where $\mu$ is the coefficient of absorption.

$\mu = 1n(2)/d_{1/2}$, where $d_{1/2}$ is the halving thickness. It is assumed that the halving thickness is inversely proportional to the density of the attenuated material:

$$d_{1/2} = C/\rho_B => \mu = 1n(2)\rho_B/C = C_1\rho_B,\ C_1 = 1n(2)/C$$

After insertion in the above formula the following result is obtained:

$$\Delta I_2(r) = \frac{\Delta I_0}{r^2} e^{-c_1 \rho_B (r-r_0)}$$

and by inserting the expression for $\Delta I_0$ the following is obtained

$$\Delta I_2(r) = \frac{E_0 \Delta m \Delta t}{r^2} e^{-c_1 \rho_B (r-r_0)}$$

By inserting the expression for $\Delta m$ the following is obtained

$$\Delta I_2(r) = \frac{4\pi r^2 \rho_J E_0 \Delta t \Delta r}{r^2} e^{-c_1 \rho_B (r-r_0)}$$

$$=>$$

$$\Delta I_2(r) = 4\pi \rho_J E_0 \Delta t e^{-c_1 \rho_B (r-r_0)} \Delta r$$

Furthermore the densities can be substituted with the result

$$\Delta I_2(r) = 4\pi J\% \rho_R E_0 \Delta t e^{-c_1 \rho_R (1+J\%)(r-r_0)} \Delta r$$

When the thickness of the spherical shell goes to zero, the formula is as follows:

$$dI_2(r) = 4\pi J\% \rho_R E_0 \Delta t e^{-c_1 \rho_R (1+J\%)(r-r_0)} dr$$

In order to find the total radiation, an integration is performed over all extremely thin spherical shells from $_0$ to r: =

$$I_2(r) = \int_{r_0}^{r} 4\pi J\% \pi_R E_0 \Delta t e^{-C_1 \rho_R (1-+J\%)(r-r_0)} dr$$

By

$$K_1 = 4\pi J\% \rho_R E_0 \Delta t,$$

and

$$K_2 = C_1 \rho_R (1+J\%),$$

the following is obtained

$$I_2(r) = K_1 \int_{r_0}^{r} e^{-K_2 (r-r_0)} dr$$

As a result:

$$I_2 = \frac{K_1}{K_2}(1 - e^{-K_2(r-r_0)})$$

- or with $K_1$ and $K_2$ inserted:

$$I_2 = \frac{4\pi J\% E_0 \Delta t}{C_1(1+J\%)}(1 - e^{-c_1 \rho_R(1+J\%)(r-r_0)})$$

## Example of measuring heavy clay and humus, respectively.

The example includes two measurings on materials being admixed 20% of earth, where both measurings involve a measuring period of 500 sec.

## Example 1 - heavy clay

The dry matter content in the earth is set to 80.2%, the reason why the measurably earth percentage is 16.0%. Based on the spectrum resulting from the measuring the following values are found:

|  | Channels | Energy range counting number | |
|---|---|---|---|
|  |  | [keV] |  |
| W87 | [75-99] | [225-297] | 11358 |
| W470 | [420-519] | [1260-1557] | 3278 |
| W200 | [140-240] | [420-720] | 0146 |

The result is

$$W470^2/W87 = 946,05$$

This number is proportional to the attenuated earth percentage. In order to convert into attenuated earth percentage, a division by a proportionality factor Ju is performed, said factor Ju being found by way of tests to be 6810, and the following value is obtained

$$\text{Attenuated earth percentage} = 13,89\%$$

Now a correction is performed for the attenuation caused by the yet unknown earth percentage, which results in a measured earth percentage of 13.89%. For this purpose the following formula is used:

$$I_2 = \frac{4\pi J\% E_0 \Delta t}{C_1(1+J\%)}(1 - e^{-C_1 \rho_R(1+J\%)(r-r_0)})$$

In this formula $I_2$ is the measured earth percentage and J% is the unknown unattenuated earth percentage. The constants are

$$E_0 = 7,19775 \times 10^{-7} \text{ m}^2/(\text{kg s})$$

$$\Delta t = 500 \text{ s}$$

$$\rho_R = 700 \text{ kg/m}^3$$

$$C_1 = 0{,}0045 \text{ m}^2/\text{kg} = 1n(2)/(d_{\frac{1}{2}}\rho_B)$$

$$r-r_0 = 1{,}50\text{m}$$

The iteration results in J% = 16,1%.

This earth percentage has not been corrected for humus. The integral W200 is known and the following number is found

$$W200/W470 = 3{,}095$$

This number is inserted in the correction function for humus:

$$HK\frac{W200}{W470} = 1 + H - \frac{H}{e^{F(\frac{W200}{W470}-S)}+1}$$

where
H = 0,35
F = 27
S = 3,4875

The correction function for humus is shown in Fig. 8. When the present value is inserted, the result is:

$$HK(3{,}095) = 1+0{,}35-\frac{0{,}35}{e^{27(3{,}095-3{,}4875)}+1} = 1{,}000$$

This function value is multiplied with the earth percentage, which in this case causes no change as the earth type is heavy clay.

Then the earth percentage is 16,1%.

This is very close to the admixed amount when a correction for water content is performed.

Example 2 - humus.

In this case 20% of humus has been admixed. The dry matter content in the earth sample was 68.5% which corresponds to a measurable earth percentage of 13.7%. The spectrum of this measuring appears from Fig. 2. The counting numbers are:

| W87 | 10202 |
|------|-------|
| W470 | 2499 |
| W200 | 9472 |

The channel numbers and energies are as stated in Example 1.

$$W470^2/W87 = 612{,}1$$

$$612{,}1/Ju = 612{,}1/6810 = 0{,}089 = 8{,}99\%$$

The correction for attenuation is found by iteration to 0,099 = 9,9%.

W200/W470 = 9472/2499 = 3,790 results in

$$HK(3,790) = 1+0,35 - \frac{0,35}{e^{27(3,790-3,4875)}+1} = 1,350$$

It should be noted that a full reflection of humus is given here. The function value is multiplied with the resulting earth percentage:

1,350 x 9,9% = 13,4%, which deviates 0,3% from the true value.

**Claims**

1. A method of detecting the earth content in agricultural crops, such as beets mixed with earth, by measuring the $\gamma$-radiation of the earth, the counting number of the measured $\gamma$-radiation being corrected for differences in different types of earth, having different contents of potassium, uranium and thorium, **characterised** in that the counting number is corrected for the content in the earth of humus by only compensating for the content of potassium in the earth.

2. A method as claimed in claim 1, **characterised** in that the correction for the content of potassium is performed by the counting number Int of the measured $\gamma$-radiation being corrected for by the factor K/overK, where Int is the counting in almost the entire energy range, K is the counting in the range containing the highest amount of potassium, and overK is the counting in the energy range thereabove.

3. A method as claimed in claim 1, **characterised** in that a correction is performed by the factor $W470^2/W87$, where W470 is the counting in the range 1260 to 1557 keV, and W87 is the counting in the range 225 to 297 keV.

4. A method as claimed in one or more of the preceding claims, **characterised** in that a further correction is performed for types of humus by means of a step function having a soft transition.

5. A method as claimed in claim 4, **characterised** in that the step function having a soft transition is of the formula

$$f(x) = 1 + h - h/(\exp(a(x - x_0)) + 1),$$

where h is the step size, $x_0$ is the point of change, and a is a constant determining the narrowness of the change range.

**Patentansprüche**

1. Verfahren zum Nachweis des Erdgehalts in landwirtschaftlichen Feldfrüchten, wie z. B. in mit Erde vermischten Rüben, durch Messen der $\gamma$-Strahlung der Erde, wobei die zählrate der gemessenen $\gamma$-Strahlung bezüglich Differenzen zwischen verschiedenen Bodenarten mit unterschiedlichen Gehalten an Kalium, Uran und Thorium korrigiert wird, dadurch gekennzeichnet, daß die Zählrate bezüglich des Humusgehalts der Erde korrigiert wird, indem nur ein Ausgleich für den Kaliumgehalt in der Erde vorgenommen wird.

2. Verfahren nach Anspruch 1, dadurch gekennzeichnet, daß die Korrektur für den Kaliumgehalt dadurch erfolgt, daß die Zählrate Int der gemessenen $\gamma$-Strahlung durch den Faktor K/überK korrigiert wird, wobei Int die Zählrate nahezu im gesamten Energiebereich, K die Zählrate im Bereich, der den höchsten Kaliumanteil enthält, und überK die Zählrate im darüberliegenden Energiebereich ist.

3. Verfahren nach Anspruch 1, dadurch gekennzeichnet, daß die Korrektur durch den Faktor $W470^2/W87$ erfolgt, wobei W470 die Zählrate im Bereich von 1260 bis 1557 keV und W87 die Zählrate im Bereich von 225 bis 297 keV ist.

**4.** Verfahren nach einem oder mehreren der vorstehenden Ansprüche, dadurch gekennzeichnet, daß eine weitere Korrektur bezüglich Humusarten mittels einer Treppenfunktion mit weichem Übergang ausgeführt wird.

**5.** Verfahren nach Anspruch 4, dadurch gekennzeichnet, daß die Treppenfunktion mit weichem Übergang durch die Formel

$$f(x) = 1 + h - h/(\exp(a(x-x_0)) + 1)$$

gegeben ist, wobei h die Schrittgröße, $x_0$ der Änderungspunkt und a eine Konstante ist, welche die Schmalheit des Änderungsbereichs festlegt.

## Revendications

**1.** Procédé de détection de la teneur en terre de produits de récolte agricole tels que des betteraves mélangées avec de la terre, par mesure du rayonnement $\gamma$ de la terre, le nombre de comptage du rayonnement $\gamma$ mesuré étant corrigé pour les différences entre les types de terre différents, ayant des teneurs différentes en potassium, uranium et thorium, **caractérisé** en ce que le nombre de comptage est corrigé pour la teneur dans la terre en humus uniquement par compensation de la teneur en potassium dans la terre.

**2.** Procédé selon la revendication 1, **caractérisé** en ce que la correction de la teneur en potassium est effectuée par correction du nombre de comptage Int du rayonnement $\gamma$ mesuré par le facteur K/overK, dans lequel Int est le comptage dans presque toute la bande d'énergie, K est le comptage dans la bande contenant la quantité de potassium la plus élevée, et overK est le comptage dans la bande d'énergie située au-dessus.

**3.** Procédé selon la revendication 1, **caractérisé** en ce qu'une correction est effectuée à l'aide du facteur W470[2]/ W87, dans lequel W470 est le comptage dans la bande comprise entre 1260 et 1557 keV, et W87 est le comptage dans la bande comprise entre 225 et 297 keV.

**4.** Procédé selon au moins l'une des revendications précédentes, **caractérisé** en ce qu'une correction supplémentaire est effectuée pour les types d'humus au moyen d'une fonction échelon comportant une transition douce.

**5.** Procédé selon la revendication 4, **caractérisé** en ce que la fonction échelon comportant une transition douce est de la formule :

$$f(x) = 1 + h - h/\exp(a(x - x_0)) + 1),$$

dans laquelle h est la taille d'échelon, $x_0$ est le point de changement, et a est une constante déterminant l'étroitesse de la plage de changement.

Fig. 1

17.2% SUGAR
14.7% EARTH

DATATRANS-
MISSION

EP 0 739 484 B1

Fig. 2

Fig. 3

1. Sandblast clay
2. Humus/clay
3. Sand
4. Sand
5. Humus
6. Clay
7. Heavy clay
8. Sand
9. Clay
10. Sand/humus
11. Sand
12. Sandblast clay
13. Sandblast clay
14. Clay
15. Sandblast clay
16. Clay
17. Heavy clay
18. Sand with salt
19. Sandblast clay
20. Sandblast clay

△ Int
⊠ Int*K/over K

Test No.

EP 0 739 484 B1

Test No. 2

*Fig. 4*

Test No. 7

Fig. 5

Test No. 15

Fig. 6

Fig. 7

1. Sandblast clay
2. Humus/clay
3. Sand
4. Sand
5. Humus
6. Clay
7. Heavy clay
8. Sand
9. Clay
10. Sand/humus
11. Sand
12. Sandblast clay
13. Sandblast clay
14. Clay
15. Sandblast clay
16. Clay
17. Heavy clay
18. Sand with salt
19. Sandblast clay
20. Sandblast clay

△ Int

⊠ Int*K/over K

✚ Corrected

EP 0 739 484 B1

Fig. 8